# EUROPEAN PATENT APPLICATION

(11) **EP 0 692 475 A1**
(43) Date of publication of application: **17.01.1996**
(21) Application number: 94910067.1
(22) Date of filing: 23.02.1994
(51) Int. Cl.: C07D 219/06, C07H 5/06, A61K 31/435

(54) **N-METHYL-N-/$g(a),$g(D)-GLUCOPYRANOZIL/AMMONIA-2-/ACRIDON-9-ON-10-YL/ACETATE/CYCLOPHERONE, WITH INTERFERON PRODUCING, ANTI-VIRAL (INCLUDING ANTI-HIV), ANTI-PARASITIC, ANTI-PROMOTER AND RADIOPROTECTIVE PROPERTIES**

(30) Priority: 01.04.1993 RU 9317260
(71) Applicant: LIMITED LIABILITY PARTNERSHIP "POLYSAN", St-Petersburg, 198207 (RU)
(72) Inventor: CHIZHOV, Novomir Pavlóvich, St-Petersburg, 195030 (RU); KUPCHINSKY, Roald Antonovich, St-Petersburg, 196240 (RU); ALEXEEVA, Ljudmila Evgenievna, St-Petersburg, 1931152 (RU); KOVALENKO, Alexey Leonidovich, St-Petersburg, 194295 (RU); BORISOVA, Margarita Aleeevna, St-Petersburg, 198205 (RU)
(74) Representative: Weisert, Annekäte, Dipl.-Ing. Dr.-Ing.
(86) International application number: PCT/RU94/00032
(87) International publication number: WO 94/22837

(57) **Abstract**

The application concerns a biologically active compound obtained by chemical synthesis and belonging to the class of heterocyclic compounds (specifically, the secondary acridones), namely: N-methyl-N-/α,Δ-glucopyranozil/ammonia-2-/acridon-9-on-10-yl/acetate of formula (I) with interferon producing, anti-viral (including anti-HIV), anti-parasitic, anti-promoter and radioprotective properties.

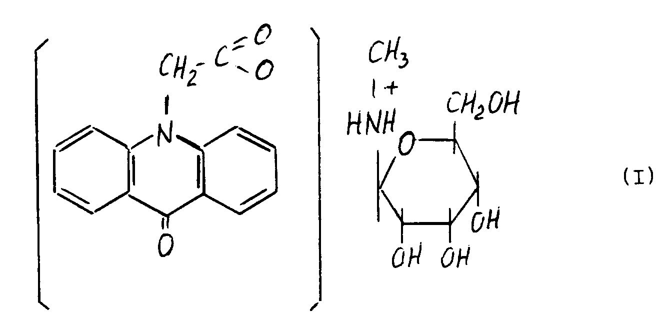

## Description

### TECHNICAL FIELD

The invention belongs to medicine, namely bioactive substances manufactured by chemical techniques. It is a new derivative of acridanones of general formula /I/ 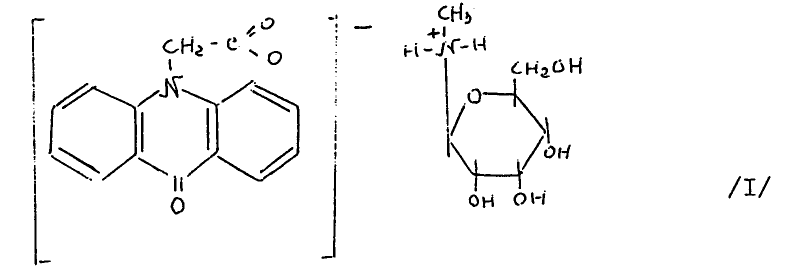
possessing a wide bioactivity range : interferonogenic, anti-viral, including anti-HIV, antiparasitic, antipromotive, radioprotective.

### PRECEDING TECHNICAL LEVEL

There have been compounds of acridanone class, occurring in nature, as well as their modifications by chemical synthesis, exhibiting antitumoural and antiviral activities /patent of Japan N 64-40426 cl. A 61 K 31/47, C 07 D 219/06, 491/052 of 10.02.89, patent of Japan N 63-310826 cl. A 61 K 31/435, C 07 D 219/06, 491/052 of 19.12.88/, along with a series of native compounds of the same class, featuring antiprotozoal and antiherpetic activities /patent of the USA N 4,244,954 cl. A 61 K 31347 of 13.01.78, Claim EP N 110298 cl. A 61 K 31/47 of 13.06.84/.

There has also been in evidence a structural analogy of cycloferone, namely 10-carboxymethyl-9-acridone dimethylaminoethylether chlorhydrate, of general formula /2/ 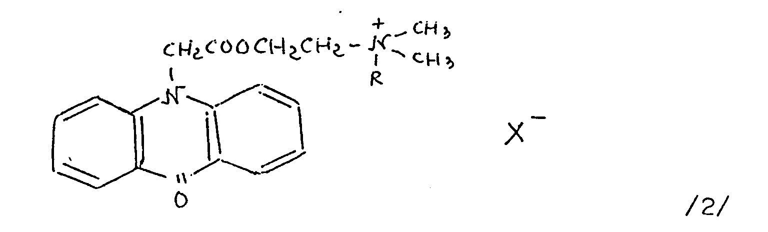
where R is hydrogen capable of interferonogenic activity and distinguished from cycloferone by a substitute in position 10 of acridone cycle /patent of Poland N 139805 cl. C 07 D 219/16 of 31.07.87/. The abovementioned substance features a lower interferonogenic activity as compared to that covered by this claim, with the same toxicity level.

### INVENTION COVERAGE

The aim of the invention is to obtain a new acridanone-derivative possessing a wide range of biological activity, a low toxicity and superior chemical-therapy evidence.

The aim is attained with the compound of general formula /I/, obtained through the interaction of the compound of general formula /3/ 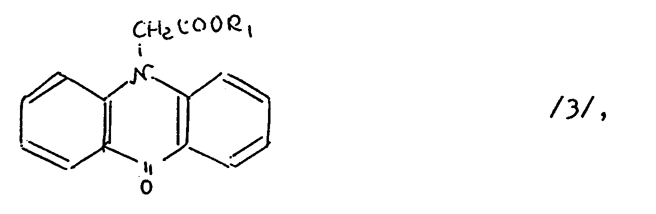
where R₁ is hydrogen, alkyl group, metal or ammonium cation, with derivatives of α , D - glucopyranosilamine of general formula /4/ 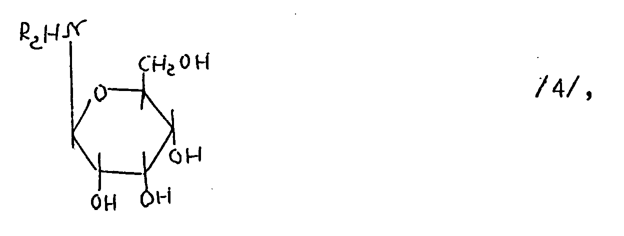
where R₂ is methyl group.

CASE 1. 99,6 g of N-methyl-N-/α,D-glucopyranosilamine/ are dissolved in 200 ml of distilled water, portions of 125 g of carefully fragmented 2-/acridone-9-on-10-yl /acetic acid added while stirring, with further stirring at room temperature until completely dissolved, and then 1.000 ml of ethyl alcohol added. The precipitate is filtered, washed in the 100 ml-ethyl alcohol filter and dried for 1 hour at 60°C. 224 g /100%/ of N-methyl-N-/α,D-glucopyranosil/-ammonium-2-/acridone-9-on-10-yl /acetate/cycloferone/ are thus obtained in yellow crystals, on being re-crystallized from low-grade alcohols possessing the melting point at 129-132°C.

C₂₂H₂₆N₂O₈

| | | | |
|---|---|---|---|
| Calculated, % | C - 58,74 | H - 5,83 | N - 6,73 |
| Found, % | C - 58,52 | H - 5,64 | N - 6,81 |

CASE 2. 276 g of sodium 2-/acridone-9-on-10-yl /acetate are dissolved while stirring in 400 ml of distilled water, then concentrated hydrochloric acid added to pH=3, the precipitated 2-/acridone-9-on-10-yl /acetic acid filtered, washed in 100 ml of distilled water filter and recrystallized from dimethylformamide-water mixture /3:1/. The 2-/acridone-9-on-10- yl/acetic acid precipitate is dried for 3 hrs at 105°C, then proceding as indicated in Case 1. The gain is 398 g /89%/ of N-methyl-N-/α,D/glucopyranosil/ammonium-2/acridone-9-on-10-yl /acetate/cycloferone, with the melting point on being recrystallized from low-grade alcohols at 129-132°C.

CASE 3. 282 g of 2-ethyl-/acridone-9-on-10-yl /-acetate are suspended in 500 ml of 20% water solution of sodium hydroxide, brought to the boiling point and stirred while boiling until the precipitate is completely dissolved ; then the reacting body is cooled to room temperature, and concentrated hydrochloric acid added to pH=3,0. The precipitate 2-/acridone-9-on-10-yl/acetic acid is filtered, washed in 300 ml-water filter and recrystallized as shown in Case 2. Further actions are similar to Case 1.

The gain is 381 g /86%/ N-methyl-N-/α,D-glucopyranosil/ammonium-2/acridone-9-on-10-yl /acetate/cycloferone/, on recristallization from lower-grade alcohols possessing the melting point at 129-132°C.

Physico-chemical characteristics of cycloferone are shown in Table I.

N-methyl-N-/α,D/glucopyranosil/ammonium-2-/acridone-9-on-10-yl /acetate/cycloferone exerts no mutagenic, terratogenic, embryotoxic or allergic effects.

### THE BEST PERFORMANCE MODE

Medical and biological characteristics of N-methyl-N-/α, D-glucopyranosil/ammonium-2-/acridone-9-on-10-yl/acetate/ cycloferone have been investigated in experiments with animals, showing a broad range of biologival activity featured by the claimed substance, along with its low toxicity, good endurance characteristics and a high therapeutic-efficiency evidence.

CASE A. Acute toxicity of cycloferone was studied in ristal-strain mongrel white mice weighing 18-20 g.

Intravenal and intramuscular injections were given of 2000 mg/kg and less in two stages. The follow-up period was 14 days. The lethal dose /LD50/ was calculated with Kerber technique. Findings from the acute toxicity studies are shown in Table 2.

CASE B. Interferonogenic Activity.

Universally known is the ability of a low-molecular fluorenone-thylorone class compound to induce generation of interferone /N.P.Chizhov, F.I.Ershov, M.K.Indulen,- "Osnovy eksperi mental'noi khimioterapii virusnykh infektsii" (Fundamentals of experimental chemical therapy for viral infections), Riga, 1988 p.89). The interferone-induction effect is exerted by low-molecular acridanone-class compounds, for example, 10-carboxymethyl-9-acridone dimethylaminoethyl ether chlorhydrate /patent of Poland N 139805 cl. C 07 D 215/16 of 31.07.87/.

The interferone-inducing activity of cycloferone was studied in mice, animal and human cellular cultures.

To BALB mice weighing 10-12 g, single hypodermic injections of 200 mg/kg cycloferone and 50 mg/kg thylorone were given. In predetermined time intervals interferone titers were defined in the blood in conventional techniques on homologous cells α-929 grown in 96-cavity in a CO₂-containing thermostate. The test-virus was the encephalomyocarditis virus. Comparison data of interferone-inducing activity exhibited by cycloferone, thylorone and cycloferone structural prototype-10-carboxymethyl-9-acridone dimethylaminoethyl ether chlorhydrate are shown in Table 3.

As can be seen from the data presented, cycloferone induces generation of early /2-8 hrs/ interferone in high titers. The peak of interferone production in the case of cycloferone exceeds interferone content in mice serum in response to thylorone injection 1000 times, and to the structural prototype - 640 times. The superior interferone-inducing effect of cycloferone has also been shown in cellular cultures.

With BALB mice, splenic-and peripheral-blood lymphocytes were isolated and induced with cycloferone and thylorone. The initial concentration was 5 x 10⁶c/ml. The dynamism of interferone accumulation was studied in the cultural fluid of lymphocytes grown on plastic panels in the presence of CO₂.

Interferone titers in splenocytes and blood cells on administration of cycloferone and thylorone are shown in Table 4.

The findings presented in Table 4 indicate that cycloferone induces significantly higher interferone levels in a mixed splenic peripheral-blood lymphocyte culture than does thylorone.

Supporting evidence has also been found for interferonogenic activity of cycloferone on human blood culture. For this purpose human peripheral-blood lymphocytes were obtained by separating the donor-blood leucocyte body. The lymphocyte concentration was 2 x 10⁶ c/ml. The cells were grown in 24-cavity plastic panels. Lymphocyte induction was performed with cycloferone and thylorone to concentration of 600 and 200 mg/ml respectively. Interferone content in the cultural fluid was evaluated by titration on diploid human cells M-19. Acting as test-virus was the virus of vesicular stomatitis. Interferone specimens were neutralized with conventional α-9 and β-9 human interferone antisera with international human interferone standards used for reference.

Data of interferone induction in human lymphocytes are shown in Table 5.

As indicated in Table 5, cycloferone induces interferone production in human peripheral-blood lymphocyte cultures at a rate 90 times higher than thylorone.

CASE C. Cycloferone Antiviral Activity.

CASE 1C. A high activity of N-methyl-N-/α,D-glucopyranosil/ammonium-2-/acridone-9-on-10-yl/acetate/cycloferone/ was demonstrated in experimental mice injection with vernal encephalitis virus /VE/.

At present only inactivated vaccines of low effectiveness and requiring annual vaccinations due to rapidly decreasing resistance are applied in the treatment strategies. No other means are available in medical practice /Smorodintsev A.A., Dubov A.V.-"Kleshchevoi entsefalit i ego profilaktika" (Vernal encephalitis and preventive vaccination, M., 1986).

To estimate cycloferone effectiveness against VE, ABSETTAROV strain of VE virus was used. The virus titer in intracerebral infection was 7,0-8,0 lg LD₅₀, in subcutaneous infection - 5,0 lg LD₅₀. The experiment was carried on with non-line white mice weighing 12-14 g, using hypodermic injections of VE virus.

Cycloferone was dissolved in salt solution, and hypodermic injections were given in test group. The controls had hypodermic injections of salt solution. The follow-up period was 21 day. The criteria for cycloferone effectiveness were survival and average life of the tests, as compared to the controls. Findings from the experimental studies are presented in Table 6.

The data testify to distinct protective effects of the prepation. Additionally, special examinations indicated that no VE virus was evident in the brain of the mice infected with cycloferone 4 and 7 days after injections, while the controls demonstrated the virus reproduced in high titers.

CASE 2C. Cycloferone activity has been verified against the human immunodefficiency virus (HIV). Only one medication has been permitted for application in medical practice todate - the one used in AIDS treatment. This is a nucleoside-based azidothymidine (retrovir, zidovudine), featuring such significant disadvantages as high toxicity and rapid generation of its virus strains. Besides, it can not guarantee survival, only a prolonged AIDS patient's life. /Chizhov N.P.//Antibiotiki i khimioterapiia. 1991.-vol.38.-N 4.-pp 38-41/.

To evaluate cycloferone effectiveness against human immunodefficiency virus (HIV) reproduction, line U-937 monocyte-cell culture was employed. The cells were grown at the RMU /Russian Medical University/ in tube medium - 1640 containing 20% calf foetal serum with the end concentration of 0.5-0.7 x 10⁶ c/ml. Prior to introducing the specimen in the supporting medium, the cellular suspension was infected with a cultural-fluid concentrate, thus feeding the HIV-producing HTA-41 lines isolated from the cells. The cells were then incubated for 3-4 days at 37°C with subsequent replacement of nutrient medium. On the 5th and 7th day after infection, the presence of virus-specific antigene was established through indirect immunofluorescence reaction using HIV-IG's.

Findings from the evaluation of cycloferone's inhibiting effect on HIV-antigene expression in HIV-inducing line U-937 monocytic cells, as compared to azidothymidine, are shown in Table 7.

Thus, cycloferone in almost 1000-times lower concentrations than azidothymidine , exerts the same effect on HIV-antigene expression in monocytic cell culture.

CASE D. The antiparasitic activity of cycloferone was studied on an experimental-chlamidiosis model in comparison with tetracycline hydrochloride - the traditional medication for this human zymotic pathology. /M.D.Mashkovskii "Lekarstvennye sredstva" (Medications), M., Meditsina, 1987, pt.2, p.221/. No interferone inductions were previously applied in the treatment of chlamidiotic infections. Application of tetracycline-series antibiotics against chlamidiotic infections requires a prolonged course of treatment and is restricted by their toxicity and rapid growth of resistant pathogene forms.

The experiment used C.trachomatis pathogene in non-line white mice weighing 16-18 g, injected with an infected-material suspension. In the test groups, cycloferone was administered 24 hours after infection in one stage of 60 mg/kg, and in two steps with a 96-hour interval. A single tetracycline hydrochloride was injected to a conventional schedule for 5 days in 100 mg/kg.

The controls had salt-solution injections.

Effectiveness evidence was evaluated by cytoscopic examination with stained smears - imprints of lymph nodes and the lung-mediating pathogenic multiplication.

Comparison data of the outcome of cycloferone and tetracycline treatment for chlamidiosis are given in Table 8. The comparison data obtained indicate a more clearly defined therapeutic effect of cycloferone, the effectiveness index being 2-4 times higher than in the tetracycline-treated animal group.

CASE E. Antipromotive Activity.

Cancerogenesis has been known as a multi-stage process involving initiation, promotion and progress. The former two stages of induce primary neoplasms, while progress means further evolution of neoplasm. The substances exhibiting antipromotive activity can be regarded as potential anticancerogens. The studies were performed on BALB/c mice-females weighing 25,0 g. The comparison used a fluorenone-class thylorone-synthesis preparation.

The specimens were administered daily in optimal doses for 6 days.6 hours after the last injection the animals were killed, a section of the lung and liver cut out, submerged in 199 medium, and cellular cohesion measured. The controls were given physiological solution.

The measurements were performed with a micro-manipulator using E.A.Modianova-modified Kuman technique /Modianova E.A., "Voprosy onkologii", 1973, vol.19., N 6, pp.83-88/.

For each experimental point, cellular cohesion was measured in 4-5 mice. A segment was examined of each of the mice, measuring 10-30 cells. Findings from the evaluation of antipromotive activity are shown in Tables 9 and 10.

Thus, with cycloferone administration, cohesion in hepacytes increases twofold, with thylorone indices the same as in the controls.

As is demonstrated by data in Table 10, cycloferone increases cellular cohesion in lung alveoli 1,2 times, as compared to thylorone and the controls.

Studies of cycloferone's antipromotive activity indicate that cycloferone behaves as a classical adhesion factor, doubling cellular cohesion in the liver and making it 1.5 higher in the lung alveoli /Modianova E.A., Bocharova O.A., Malenkov A.G., Coll. "Problemy experimental'nogo kantserogeneza" (Problems of Experimental Cancerogenesis), Lyonnes, 1983, N 51, pp.135-139).

CASE F. Radioprotective Activity.
Radioprotective activity of cycloferone was studied in BALB/c mice weighing 26.7 ± 1.84 g on the average. The mice were exposed to ⁶⁰Co irradiation in the range of 7.5 + 9.0 Grey. Cycloferone was administered in a single dose of 300 mg/kg through hypodermic injections 2 hours before the exposure. The controls were given physiological-solution injections. The follow-up period was 14 days.

The effectiveness criteria were based on survival evidence. Findings from the experimental studies are presented in Table II.

Thus, single subcutaneous administrations of cycloferone 2 hours before exposure provide protection of 70% mice against ⁶⁰Co of 8.7 Gy, and 50-60% against 8.0-8.5 Gy and 40% - against the maximum dose of 9.0 Gy, testifying to cycloferone's high radioprotective effects.

### COMMERCIAL APPLICATIONS.

The abovedescribed indicates that the designers succeeded in manufacturing a new acridone-class compound featuring a wide range of biological activity, including those previously unknown for the compound class (antiparasitic, radioprotective).

Due to its low toxicity, it would be worthwhile using it as a basis to design medications for parenteral, peroral and external application.

The Claimant claims the special name CYCLOFERONE for the compound, being at present registered as a trade mark in accordance with international procedure.

**Table I**

| Parameter | | | Analytical Data |
|---|---|---|---|
| Appearance | | | Crystalline powder of yellow colour, odourless |
| Solubility | | | Readily soluble in water, poorly soluble in lower-grade alcohols, insoluble in ether, chloroform, acetone. |
| UV-spectrum 0.002% solution in water,nm | | | 210, 255, 392, 408 |
| PMR -spectrum, δ, /D₂O/ | | | 2,5/s/, 3,0-3,1/t/, 3,5-3,8/m/, 4,0/m/, 4,5/s/, 7,1-7,25/m/, 7,55-7,65/m/, 7,95-8,05/m3 |
| NMR^{I3}C-spectrum,δ, /D₂O/ | | | 35,622; 50,472; 51,761; 63,352; 68,710; 71,185; 71,391; 71,594; 115,829; 121,065; 122,686; 126,844; 135,550; 175,602; 179,532; |

| Elemental analysis | | | |
|---|---|---|---|
| C₂₂H₂₆N₂O₈ | calculated | C | 58,74 |
| | | H | 5,83 |
| | | N | 6,73 |
| | found | C | 58,52 |
| | | H | 5,64 |
| | | N | 6,81 |
| Molecular mass | | | 446,25 |
| Melting temperature °C | | | 129-132°C |

**Table 2**

| Animals | Administration Mode | LD₅₀ | Parameter |
|---|---|---|---|
| | | Cycloferone | 10-Carboxymethyl-9-acridone dimethylaminoethyl ether chlorhydrate /Patent of Poland N 139805 of 31.07.87 Cl. C07D 219/16/ -/prototype/ |
| Mice | intravenal | 400 | 400 |
| | intramuscular | 500 | 600 |

As is seen from data in the Table, cycloferone and the prototype belong to low-toxic substances in parenteral application.

**Table 3**

| Preparation | Dose mg/kg | Interferone titers in international units /IU/ml on administration /hour/ | | | | | | |
|---|---|---|---|---|---|---|---|---|
| | | 1 | 2 | 4 | 8 | 18 | 24 | 48 |
| Cycloferone | 200 | 80 | 160000 | 640800 | 8000 | 640 | 320 | 10 |
| Thylorone | 50 | <40 | <40 | <40 | 40 | 640 | 20 | <10 |
| Dimethylaminoethyl ether chlorhydrate | 400-690 | - | 100 | 1000 | - | - | - | - |
| 10-carboxymethyl-9-acridone /patent of Poland N 139805 cl. C07D 219/16 of 31.07.87/ | | | | | | | | |

**Table 4**

| Cells | Cycloferone | | | | | | Thylorone | | | | | |
|---|---|---|---|---|---|---|---|---|---|---|---|---|
| | 24hr | 48hr | 72hr | 96hr | 120hr | 144hr | 24hr | 48hr | 72hr | 96hr | 120hr | 144hr |
| Splenocytes | 2560 | 1280 | 5120 | 640 | 80 | <10 | 15 | 30 | 640 | 320 | 15 | <5 |
| Peripheral-blood lymphocytes | 2560 | 1280 | 2560 | 160 | <10 | <10 | 40 | 80 | 320 | 80 | 10 | <10 |
| T-lymphocytes | 10 | 40 | <10 | <10 | <10 | <10 | 15 | 60 | 5000 | 3600 | 960 | 5 |
| B-lymphocytes | 2500 | 1280 | 5120 | 1280 | 160 | 10 | 15 | 30 | 320 | <10 | <10 | 5 |

**Table 5**

| Preparation | Interferone Titers /U/ml/ | | | | |
|---|---|---|---|---|---|
| | 24hr | 48hr | 72hr | 96hr | 120hr |
| Cycloferone | 1280 | 40 | 80 | <10 | <10 |
| Thylorone | 15 | 60 | 120 | 30 | <15 |

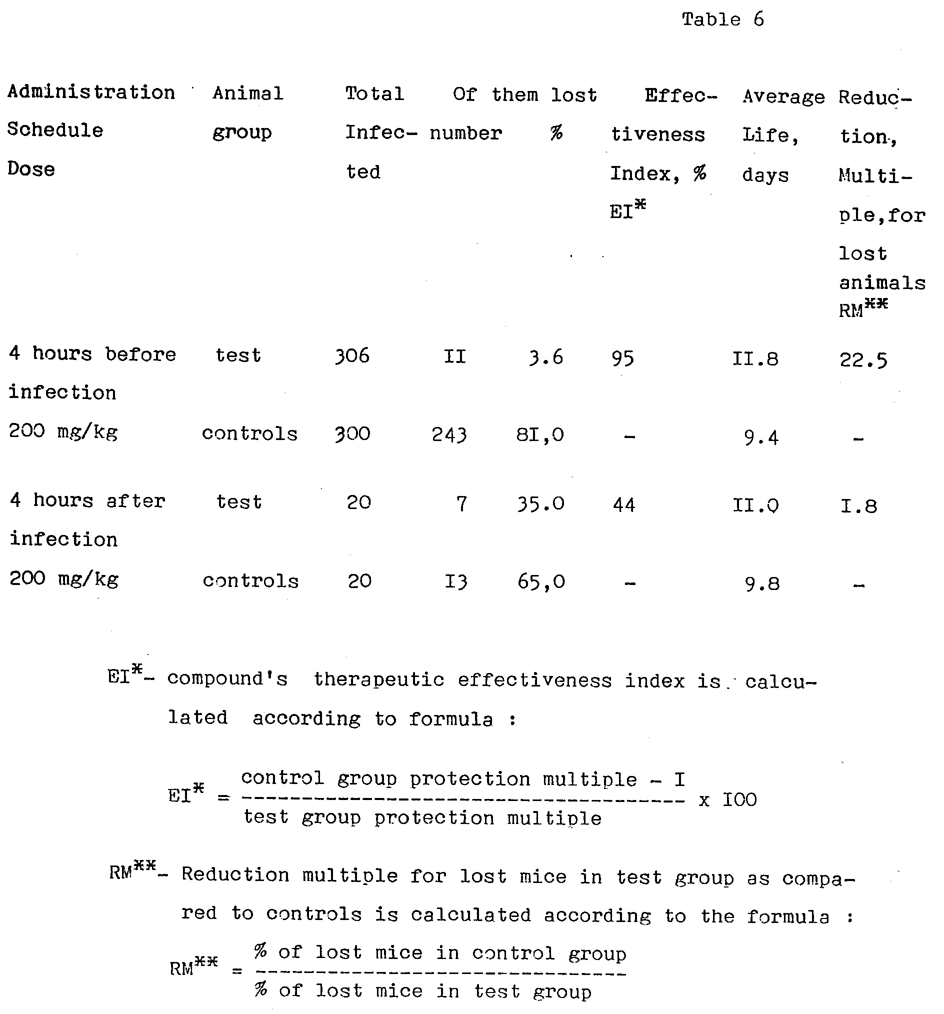

**Table 7**

| Preparation | Concentration mg/ml | Number of cells containing HIV-antigene in IIFR* in 24 hours, % | |
|---|---|---|---|
| | | 5 | 7 |
| Cycloferone | 0.09 x 10⁻³ | 5.1 | 4.8 |
| | 0.18 x 10⁻³ | 2.1 | 3.7 |
| Azidothymidine /Wellcome, Inc. | 0.08 | 6.5 | 4.7 |
| | 0.16 | 3.9 | 2.4 |
| Great Britain/ Controls | - | 35.6 | 32.4 |

| | | | |
|---|---|---|---|
| IIFR* - indirect immunofluorescence reaction | | | |

**Table 8**

| Preparation | Administration Schedule | Dose, mg/kg | Effectiveness Index | | | |
|---|---|---|---|---|---|---|
| | | | lymphnodes | | lung | |
| | | | 3days | 7days | 3days | 7days |
| Cycloferone | 24 hours after infection single | 60 | 4.7 | 1.8 | 3.6 | 3.2 |
| | 24 hours after infection, single; and in 96 hours, single | 60x2 | 4.4 | 2.6 | 3.4 | 3.9 |
| Tetracycline hydrochloride | Daily 1 x 5 | 100x5 | 0.9 | 0.6 | 1.8 | 1.0 |

**Table 9**

| Compound | Administration Mode, dose mg/kg | Hepatocytic cohesion, mg/l cell | P |
|---|---|---|---|
| Cycloferone | Intraperitoneal, 100.0 | 0.113 ± 0.017 | <0.05 |
| Thylorone | Intraperitoneal, 10.0 | 0.053 ± 0.008 | >0.05 |
| | Peroral, 200.0 | 0.076 ± 0.013 | >0.05 |
| Controls | Physiological | 0.056 ± 0.008 | - |

**Table 10**

| Preparation | Administration Schedule, dose, mg/kg | Cellular cohesion in lung alveoli, mg/kg | P |
|---|---|---|---|
| Cycloferone | Intraperitoneal, 100,0 | 0.324 ± 0.033* | <0.05 |
| Thylorone | Intraperitoneal, 10,0 | 0.289 ± 0.028 | >0.05 |
| | Oral 200.0 | 0.290 ± 0.024 | >0.05 |
| Controls | Phys. solution | 0.279 ± 0.024 | - |

| | | | |
|---|---|---|---|
| * - reliable differences | | | |

**Table II**

| Preparation | Irradiation Dose, GR | Number of animals | | Loss % | Protection % |
|---|---|---|---|---|---|
| | | Total Exposed | Lost | | |
| Cycloferone /test/ | 8.0 | 10 | 1 | 10 | 60 |
| | 8.5 | 10 | 3 | 30 | 50 |
| | 8.7 | 10 | 1 | 10 | 70 |
| | 9.0 | 10 | 6 | 60 | 40 |
| | 9.3 | 10 | 9 | 90 | 10 |
| | 10.0 | 10 | 10 | 100 | 0 |
| Phys.Solution /controls/ | 7.5 | 10 | 4 | 40 | - |
| | 7.8 | 10 | 3 | 30 | - |
| | 8.2 | 10 | 7 | 70 | - |
| | 8.5 | 10 | 8 | 80 | - |
| | 8.7 | 10 | 8 | 80 | - |
| | 9.0 | 10 | 10 | 100 | - |
| | 9.3 | 10 | 10 | 100 | - |
| | 10.0 | 10 | 10 | 100 | - |

## Claims

1. N-methyl-N-/α,D-glucopyranosil/ammonium-2-/acridone-9-on-10-yl/acetate, of formula 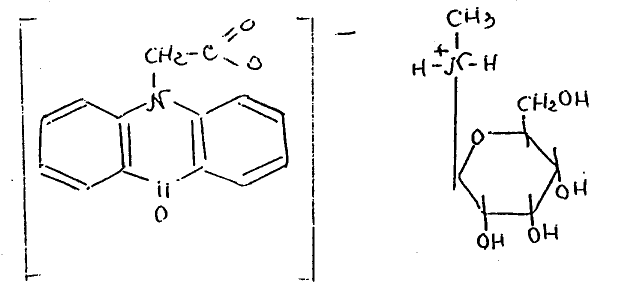 featuring interferonogenic, antiviral, including anti-HIV, antiparasitic, antipromotive and radioprotective activity.
